# EUROPEAN PATENT APPLICATION

(11) **EP 4 542 288 A1**
(43) Date of publication of application: **23.04.2025**
(21) Application number: 22946686.7
(22) Date of filing: 16.06.2022
(51) Int. Cl.: G02C 7/16

(54) **METHOD FOR DETERMINING THE POSITION OF THE CENTRE OF OCULAR ROTATION WITH RESPECT TO A LENS AND DEVICE FOR SAID METHOD**

(71) Applicant: JJ Gestión Integral S.L., 48901 Barakaldo/Bizkaia (ES)
(72) Inventor: VILLAVERDE ROSENDE, Julio Manuel, 09500 MEDINA DE POMAR/BURGOS (ES)
(74) Representative: Del Valle Valiente, Sonia
(86) International application number: PCT/ES2022/070381
(87) International publication number: WO 2023/242447

(57) **Abstract**

The invention relates to a method for determining the position of the centre of ocular rotation (1) with respect to a lens (2), said method establishing fixation points (3) of the vision of the eye (10) at two different angular measurement positions. The method further comprises measuring the angle between both positions, arranging references (6, 6a) to be aligned with the fixation point (3) in the different angular measurement positions of the eye using the visual axis (5), thereby obtaining two straight lines, measuring the distance between the references (6, 6a) when they have been aligned with the visual axis (5) and the fixation point (3), and drawing straight lines (100, 101) that form the same angle therebetween as the angular measurement positions of the eye, and pass through the ends of a first longitudinal straight line (55) that is equal to the distance between the two references (6, 6a).

## Description

### OBJECT OF THE INVENTION

The present invention relates to a method for determining the position of the centre of ocular rotation with respect to a lens in order to minimise geometric aberrations (oblique astigmatism and power error, as far as possible, transverse chromatic aberration), and to a device specifically intended for implementing said method.

### BACKGROUND OF THE INVENTION

The visual axis is defined as that which joins the fixation point with the centre of the macula on the retina. It is also called achromatic axis. In addition, the centre of ocular rotation would be approximately the point about which the eyeball rotates.

Moreover, the distance between the rear portion of a lens and the centre of ocular rotation is a piece of data of special interest when designing lenses, whether they are progressive geometry lenses or single vision lenses, and it is also a piece of data of interest when centring and adapting lenses, so that their behaviour is more comfortable and less aberrant. It is also a simple way to reduce the geometric aberrations (oblique astigmatism and power error) induced by lenses.

At present, it is not possible to determine the centre of rotation precisely on the visual axis, so the manufacture of lenses cannot take into account this very personal piece of data in each user; an estimate is simply made with a device, but it is an estimate that is not based on the positions of the visual axis, so it is not exact.

### DESCRIPTION OF THE INVENTION

The method for determining the position of the centre of ocular rotation with respect to a lens of the invention comprises the following steps:
- establishing one or two fixation points to fixate the vision of the eye to be evaluated at two different angular measurement positions, and measuring or determining the angle between said different angular measurement positions,
- arranging references to be aligned with the fixation point(s) in the different angular measurement positions of the eye using the visual axis, in turn obtaining two straight lines that form an identical angle therebetween as the angle formed by the angular measurement positions of the eye,
- determining or measuring the distance between the references when they have been aligned with the visual axis and the fixation point(s); and
- obtaining the position of the centre of ocular rotation by drawing a first longitudinal straight line that is equal to the distance between the two references, drawing a second straight line through the end of the first straight line, and drawing a third straight line through the opposite end of the first straight line forming an angle with the second straight line that is equal to that formed by the angular measurement positions of the eye, the position of the centre of ocular rotation being determined at the intersection of the second straight line and the third straight line.

Thus, the quasi-accurate determination of the subjective centre of ocular rotation is achieved, and thereby the distance from the same to any point (e.g. the rear portion of a lens) can be calculated subjectively in order to fine-tune its graduation. In addition, it also makes it possible to find out the distance from the visual axis to the temporal portion, and from the visual axis to the nasal portion, allowing the use of computerised means to draw the lines. These data, together with the graduation, wrap angle, pantoscopic angle, mounting heights and position of the visual axis, allows the manufacturer to design and manufacture the lenses, which can be either single or multifocal, with accuracy.

The invention also comprises a device for determining the position of the centre of ocular rotation with respect to a lens, comprising two eye alignment references arranged at a known distance therebetween, and placement means for placing said references laterally displaced on either side of the eye to be evaluated, ideally in a frame similar to that of spectacles. With the device, the method of the invention becomes as simple as placing the device in front of the point of fixation of the vision and rotating the head to both sides to find the alignments, measuring the rotation angles of the head, to then process the distance between the references and the angles according to the method of the invention.

### DESCRIPTION OF THE DRAWINGS

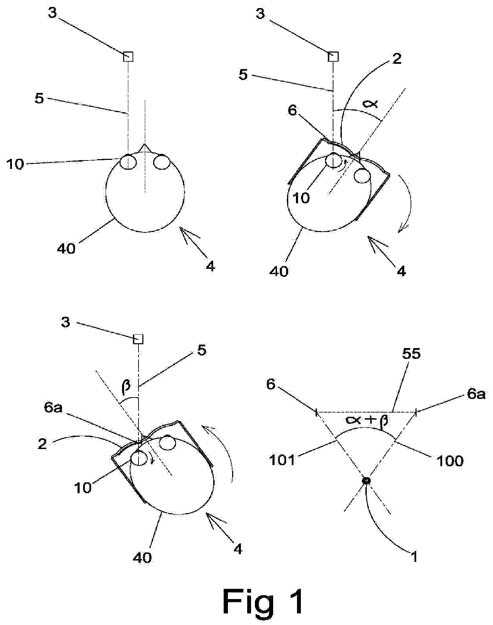

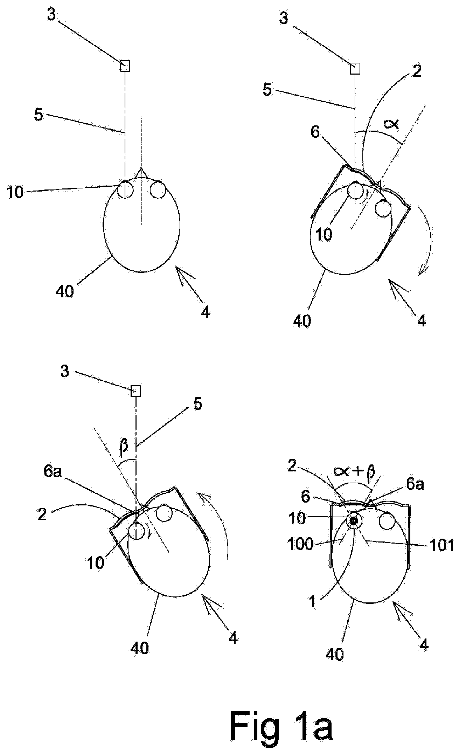

Figure 1 shows a schematic view showing the steps of the method of the invention.
Figure 1 a shows a schematic view, similar to that in figure 1, with a variant of the step of obtaining the position of the centre of ocular rotation.
Figure 2 shows a view of the template with pinhole slots, according to the invention.
Figures 3 and 4 show the placement of the template of figure 2 in a frame, on the inner side of a model lens.
Figure 5 shows a view of a frame with model lenses with diffraction marks, according to the invention.

### PREFERRED EMBODIMENT OF THE INVENTION

The method for determining the position of the centre of ocular rotation (1) with respect to a lens (2) of the invention comprises the following steps:
- establishing one or two fixation points (3) to fixate the vision of the eye (10) to be evaluated at two different angular measurement positions, and measuring or determining the angle between said different angular measurement positions,
- arranging references (6, 6a) to be aligned with the fixation point(s) (3) in the different angular measurement positions of the eye using the visual axis (5), in turn obtaining two straight lines that form an identical angle therebetween as the angle formed by the angular measurement positions of the eye,
- determining or measuring the distance between the references (6, 6a) when they have been aligned with the visual axis (5) and the fixation point(s) (3); and
- obtaining the position of the centre of ocular rotation (1) by drawing a first longitudinal straight line (55) that is equal to the distance between the two references (6, 6a), drawing a second straight line (100) through the end of the first straight line (55), and drawing a third straight line (101) through the opposite end of the first straight line (55) forming an angle with the second straight line (100) that is equal to that formed by the angular measurement positions of the eye, the position of the centre of ocular rotation (1) being determined at the intersection of the second straight line (100) and the third straight line (101).

For example, in a first variant embodiment, the method comprises:
- establishing a fixation point (3) of the vision, facing the subject (4) at rest, to establish the rest position of the visual axis (5), where as shown in figure 1 and figure 1a, said visual axis (5) and the orientation of the head (40) in this rest position are parallel,
- arranging in front of the eye (10) to be evaluated the two eye alignment references (6, 6a), laterally displaced on either side of the eye (10), said references (6, 6a) being located at a known distance,
- rotating the head (40) of the subject to one side and the eye (10) to the opposite side, until the visual axis (5), the first lateral reference (6) and the fixation point (3) are aligned, and measuring the first angle α of rotation of the head (40) with respect to the rest position,
- rotating the head (40) of the subject to the other side and the eye to the opposite side until the visual axis (5), the second lateral reference (6a) and the fixation point (3) are aligned, and measuring the second angle β of rotation of the head (40) with respect to the rest position, thereby obtaining the angle α+β between the different angular positions of measurement and
- obtaining the position of the centre of ocular rotation (1) by drawing the first longitudinal straight line (55) that is equal to the distance between the two references (6, 6a), drawing the second straight line (100) through the end of the first straight line (55), and drawing the third straight line (101) through the opposite end of the first straight line (55) forming an α+β angle with the second straight line (100), the position of the centre of ocular rotation (1) being determined at the intersection between the second straight line (100) and the third straight line (101) with respect to the references (6, 6a), which coincide with the ends of the first straight line (55).

The invention covers all possible geometrical equivalences that can be used to obtain the same result. For example, the step of obtaining the position of the centre of ocular rotation (1) could comprise (see figure 1a) drawing, in plan, the second straight line (100) so that it passes through the second reference (6a), and the third straight line (101) so that it passes through the first reference (6) and that forms an angle α+β with the second straight line (100) (since the distance between both references (6, 6a) is determined and is therefore known), and obtaining the point of intersection or crossing of the second straight line (100) and the third straight line (101), the position of the centre of ocular rotation (1) being determined at this point.

The method could also be carried out (variant not shown) by placing two fixation points located at a known distance and forming the angle α+β from the observer, and by means of two movable pinhole slots, measuring the distance at which said slots must be placed until the subject can see the first fixation point through the first slot and subsequently move the slot until the subject can see the second fixation point and, these distances and angle being known, proceeding with the step of obtaining the position of the centre of ocular rotation.

Preferably, the step of obtaining the position of the centre of ocular rotation (1) is computer-implemented, as this allows the drawing, positioning and measurement to be carried out accurately and quickly, by implementing a specific application.

For the correction of transverse chromatic aberration, this can be carried out by choosing materials with a suitable Abbe number for low lens powers or with systems combining lens doublets with different refractive indexes to achieve achromatic systems. It can also be done by means of diffraction systems through the deposition of stratified layers on the lenses with an orientation that reduces chromatic aberration.

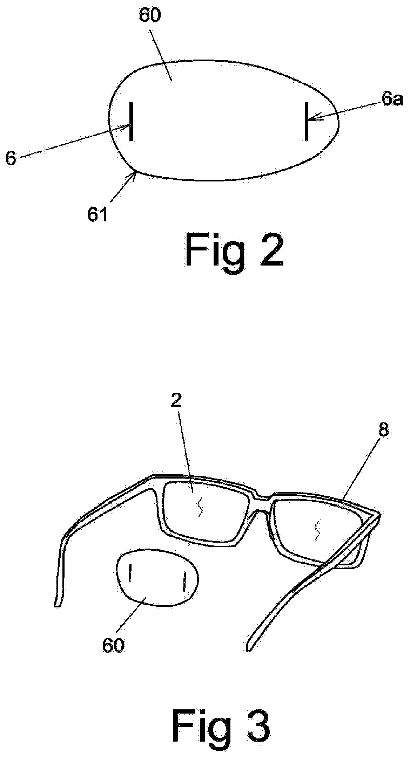

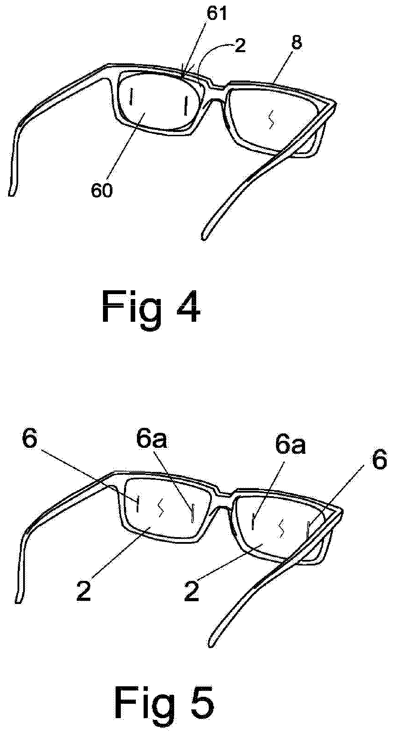

In a first variant of the invention shown in figures 2 to 4, the displaced references (6, 6a) may comprise two pinhole slots made in an opaque template (60) comprising fixing means for fixing to a frame (8) which can be placed on the head of the user and which is facing the eye to be measured. In this way, the template can be placed in whatever frame the user chooses as the final spectacle frame and adjustments can be made thereto, so that the finished spectacle will have the lens perfectly adjusted to its centre of ocular rotation. The frame (8) may comprise a model lens (2), the template (60) being arranged on the inner side (as shown in figure 4) or the outer side thereof; the fixing means for fixing the template (60) comprising edges (61) for fitting therein or mounting side pieces, not shown.

Alternatively (see figure 5), the displaced references (6, 6a) may comprise two vertically superimposed marks (diffraction marks) made on a model lens (2) arranged in a frame (8) which can be placed on the head (40) of the user and which is facing the eye to be measured, with the same advantages as in the previous case. Figure 5 shows this variant, with two model lenses (2) for both eyes, which should be evaluated separately.

Thus, the invention also comprises the implementation of a device for determining the position of the centre of ocular rotation (1) with respect to a lens (2), said method comprising two eye alignment references (6, 6a) arranged at a known distance therebetween, and placement means for placing said references (6, 6a) laterally displaced on either side of the eye to be evaluated, wherein the placement means for placing the eye alignment references (6, 6a) may comprise an opaque template (60) with two pinhole slots constituting the references (6, 6a), and wherein the template (60) has fixing means for fixing to a frame (8) which can be placed on the head (40) of the user and which is facing the eye to be measured, wherein the frame (8) comprises a model lens (2); the template (60) being placed on the front or rear face of the model lens (2); and/or wherein the placement means for placing the eye alignment references (6, 6a) comprise a frame (8) with at least one model lens (2) facing the eye to be measured; the model lens (2) comprising two vertically superimposed marks (diffraction marks) constituting the references (6, 6a).

Having sufficiently described the nature of the invention, it is stated that the description of the invention and its preferred embodiment is to be interpreted in a non-limiting manner and that it covers all the possible variant embodiments that can be deduced from the contents of this specification and the claims.

## Claims

1. A method for determining the position of the centre of ocular rotation (1) with respect to a lens (2) **characterised in that** it comprises the following steps:
- establishing one or two fixation points (3) to fixate the vision of the eye (10) to be evaluated at two different angular measurement positions, and measuring or determining the angle between said different angular measurement positions,
- arranging references (6, 6a) to be aligned with the fixation point(s) (3) in the different angular measurement positions of the eye using the visual axis (5), in turn obtaining two straight lines that form an identical angle therebetween as the angle formed by the angular measurement positions of the eye,
- determining or measuring the distance between the references (6, 6a) when they have been aligned with the visual axis (5) and the fixation point(s) (3); and
- obtaining the position of the centre of ocular rotation (1) by drawing a first longitudinal straight line (55) that is equal to the distance between the two references (6, 6a), drawing a second straight line (100) through the end of the first straight line (55), and drawing a third straight line (101) through the opposite end of the first straight line (55) forming an angle with the second straight line (100) that is equal to that formed by the angular measurement positions of the eye, the position of the centre of ocular rotation (1) being determined at the intersection of the second straight line (100) and the third straight line (101).

2. The method for determining the position of the centre of ocular rotation (1) with respect to a lens (2) according to claim 1, **which** comprises the steps of:
- establishing a fixation point (3) of the vision, facing the subject (4) at rest, to establish the rest position of the visual axis (5),
- arranging in front of the eye (10) to be evaluated the two eye alignment references (6, 6a), laterally displaced on either side of the eye (10), said references (6, 6a) being located at a known distance,
- rotating the head (40) of the subject to one side and the eye (10) to the opposite side, until the visual axis (5), the first lateral reference (6) and the fixation point (3) are aligned, and measuring the first angle α of rotation of the head (40) with respect to the rest position,
- rotating the head (40) of the subject to the other side and the eye to the opposite side until the visual axis (5), the second lateral reference (6a) and the fixation point (3) are aligned, and measuring the second angle β of rotation of the head (40) with respect to the rest position, thereby obtaining the angle α+β between the different angular positions of measurement, and
- obtaining the position of the centre of ocular rotation (1) by drawing the first longitudinal straight line (55) that is equal to the distance between the two references (6, 6a), drawing the second straight line (100) through the end of the first straight line (55), and drawing the third straight line (101) through the opposite end of the first straight line (55) forming an α+β angle with the second straight line (100), the position of the centre of ocular rotation (1) being determined at the intersection between the second straight line (100) and the third straight line (101) with respect to the references (6, 6a).

3. The method for determining the position of the centre of ocular rotation (1) with respect to a lens (2) according to claim 2, **wherein** the step of obtaining the position of the centre of ocular rotation (1) comprises drawing the second straight line (100) so that it passes through the second reference (6a), and drawing the third straight line (101) so that it passes through the first reference (6) and forms an angle α+β with the second straight line (100), and obtaining the point of intersection or crossing of the second straight line (100) and the third straight line (101), the position of the centre of ocular rotation (1) being determined at this point.

4. The method for determining the position of the centre of ocular rotation (1) with respect to a lens (2) according to claim 1, **which** comprises arranging two fixation points located at a known distance and forming the angle α+β from the observer, and by means of two movable pinhole slots measuring the distance at which said slots (6, 6a) must be placed until the subject can see the first fixation point through the first slot and subsequently move the slot until the subject can see the second fixation point and, these distances and angle being known, proceeding with the step of obtaining the position of the centre of ocular rotation.

5. The method for determining the position of the centre of ocular rotation (1) with respect to a lens (2) according to any of the preceding claims, **wherein** for the correction of the transverse chromatic aberration the following are selected:
- materials with an Abbe number suitable for low lens powers, or - combination systems of lens doublets with different refractive indexes that achieve achromatic systems, or
- diffraction systems through the deposition of stratified layers on the lenses with an orientation that reduces chromatic aberration.

6. The method for determining the position of the centre of ocular rotation (1) with respect to a lens (2) according to any of the preceding claims, **wherein** the step of obtaining the position of the centre of ocular rotation (1) is computer-implemented.

7. The method for determining the position of the centre of ocular rotation (1) with respect to a lens (2) according to any of the preceding claims, **wherein** the displaced references (6, 6a) comprise two pinhole slots made in an opaque template (60) comprising fixing means for fixing to a frame (8) which can be placed on the head (40) of the user and which is facing the eye to be measured.

8. The method for determining the position of the centre of ocular rotation (1) with respect to a lens (2) according to claim 7, **wherein** the frame (8) comprises a model lens (2), the template (60) being arranged on the inner or outer side thereof; the fixing means for fixing the template (60) comprising edges (61) for fitting therein or mounting side pieces.

9. The method for determining the position of the centre of ocular rotation (1) with respect to a lens (2) according to any of claims 1 to 4, **wherein** the displaced references (6, 6a) comprise two vertically superimposed marks made on a model lens (2) arranged in a frame (8) which can be placed on the head (40) of the user and which is facing the eye to be measured.

10. A device for determining the position of the centre of ocular rotation (1) with respect to a lens (2), **characterised in that** it comprises two eye alignment references (6, 6a) arranged at a known distance therebetween, and placement means for placing said references (6, 6a) laterally displaced on either side of the eye to be evaluated.

11. The device for determining the position of the centre of ocular rotation (1) with respect to a lens (2) according to claim 10, **wherein** the placement means for placing the eye alignment references (6, 6a) comprise an opaque template (60) with two pinhole slots constituting the references (6, 6a), and wherein the template (60) has fixing means for fixing to a frame (8) that can be placed on the head (40) of the user and which is facing the eye to be measured.

12. The device for determining the position of the centre of ocular rotation (1) with respect to a lens (2) according to claim 11, **wherein** the frame (8) comprises a model lens (2); the template (60) being placed on the front or rear face of the model lens (2).

13. The device for determining the position of the centre of ocular rotation (1) with respect to a lens (2) according to claim 10, **wherein** the placement means for placing the eye alignment references (6, 6a) comprise a frame (8) with at least one model lens (2) which is facing the eye to be measured; the model lens (2) comprising two vertically superimposed marks constituting the references (6, 6a).
